# EUROPÄISCHE PATENTSCHRIFT

(11) **EP 3 697 336 B1**
(45) Veröffentlichungstag und Bekanntmachung des Hinweises auf die Patenterteilung: **08.12.2021**
(21) Anmeldenummer: 18799445.4
(22) Anmeldetag: 16.10.2018
(51) Int. Cl.: A61C 19/05, G16H 50/50

(54) **VERFAHREN ZUR ERMITTLUNG UND VISUALISIERUNG VON ZAHNSTELLUNGEN UNTER EINWIRKUNG VON BEISSKRÄFTEN**
METHOD FOR DETERMINING AND VISUALIZING TOOTH POSITIONS UNDER THE ACTION OF BITING FORCES
PROCÉDÉ POUR DÉTERMINER ET VISUALISER LA POSITION DE DENTS SOUS L'EFFET DE FORCES OCCLUSALES

(30) Priorität: 20.10.2017 DE 102017124580
(43) Veröffentlichungstag der Anmeldung: 26.08.2020
(73) Patentinhaber: SICAT GmbH & Co. KG, 53175 Bonn (DE)
(72) Erfinder: HANSSEN, Nils, 53125 Bonn (DE)
(74) Vertreter: Braun-Dullaeus Pannen Emmerling Patent- & Rechtsanwaltspartnerschaft mbB
(86) Internationale Anmeldenummer: PCT/EP2018/078231
(87) Internationale Veröffentlichungsnummer: WO 2019/076888

(56) Entgegenhaltungen:
- US-A1- 2006 275 736
- ISIS A. VENTURINI P. POIATE ET AL: "Three-Dimensional Stress Distribution in the Human Periodontal Ligament in Masticatory, Parafunctional, and Trauma Loads: Finite Element Analysis", JOURNAL OF PERIODONTOLOGY., Bd. 80, Nr. 11, 1. November 2009 (2009-11-01), Seiten 1859-1867, XP055534927, US ISSN: 0022-3492, DOI: 10.1902/jop.2009.090220

## Beschreibung

Die Erfindung betrifft ein Computerimplementiertes Verfahren zur Darstellung einer Zahnstellung unter der Einwirkung vom Beißkräften. Dabei werden zunächst ein erster Satz dreidimensionaler Oberflächendaten von zumindest einem Abschnitt der Zahnreihe des Oberkiefers und ein zweiter Satz von dreidimensionalen Oberflächendaten von zumindest einem gegenüberliegenden Abschnitt der Zahnreihe des Unterkiefers angefertigt. Diese beiden Datensätze enthaltend dreidimensionale Oberflächendaten werden dann zur Erzeugung eines digitalen Oberflächenmodells vereinigt.

Ein solches Verfahren ist beispielsweise aus DE 10 2012 214 470 A1 bekannt. Aus der DE 10 2016 103 320 A1 ist ein System zur intraoralen Messung von Kieferverlagerungen bekannt. Solche digitalen Modelle der Zähne werden in der Zahnheilkunde zunehmend zur Diagnose und im Rahmen der Herstellung von Zahnersatz verwendet. Dabei wird ein Satz der Oberflächendaten von zumindest einem Abschnitt einer Zahnreihe insbesondere mit einer digitalen Intraoralkamera als dreidimensionales Oberflächenmodell erfasst. Dabei sind im Falle von Aufbissbehelfen und von Zahnersatz insbesondere die Kontaktpunkte respektive die Kontaktflächen der Zähne mit den entsprechenden Zähnen des Gegenkiefers und die beim Beißen auftretenden Kräfte von Bedeutung und geraten immer mehr in den Fokus des Interesses.

Dokument US 2006/275736 A1 offenbart ein Verfahren zur Modellierung einer Zahnstellung unter Einwirkung von Beißkräften, wobei die Oberflächendaten der Zahnreihe des Oberkiefers und des Unterkiefers angefertigt werden, wobei erste Oberflächendaten den Oberkiefer und zweite Oberflächendaten zeigen den Unterkiefer zeigen wobei Druck oder Kraft auf einen Zahn im Mund eines Patienten ausgeübt wird, während der Betrag der Verschiebung oder Drehung des Zahns gemessen wird, und wobei unter Berücksichtigung der Bewegungen die Eigenschaften des Ligaments der Zähne in der Software berücksichtigt ist, sodass der modifizierte Zustand der Zähne des Oberkiefers und Unterkiefers anhand von Spannung oder Druck simuliert wird.

Aus dem Stand der Technik ist es bekannt, mit einer Intraoralkamera Oberflächendaten vom Oberkiefer und vom Unterkiefer aufzunehmen und diese dann mittels einer Aufnahme der Zahnaußenflächen im Schlussbiß, mithin einer Bukkalaufnahme, räumlich zuzuordnen. Durch die so ermittelte räumliche Zuordnung können statische Kontaktpunkte und Kontaktflächen mit den jeweiligen Zähnen im Gegenkiefer berechnet und zur Anzeige gebracht werden.

Nachteilig an dieser Vorgehensweise ist, dass mögliche Einzelbewegungen der Zähne bei der Zuordnung von Unterkiefer zu Oberkiefer mittels der Bukkalaufnahme nicht berücksichtigt werden. Wegen der zwischen den Kiefern wirkenden Beißkräfte werden die Einzelzähne in ihren Ligamenten bewegt. Zudem führen die in den Kiefern wirkenden Muskelkräfte insbesondere im Unterkiefer zu einer nicht-rigiden Verformung. Durch die im Stand der Technik getätigte Annahme starrer Verhältnisse mithin der Rigidität wird die Zuordnung von Unterkiefer zu Oberkiefer verfälscht, so dass die Bewegungen einzelner Zähne bei Kontakt unter Kraft und Verformungen insbesondere des Unterkiefers nicht korrekt vorhergesagt werden können. Das führt dazu, dass die Kontaktpunkte auf den Okklusionsflächen nicht korrekt ermittelt und angezeigt werden können, so dass Zahnersatz nur in verminderter Qualität und Passung hergestellt werden kann.

Weiterhin ist es bekannt, eine Sensorfolie zwischen die Zähne von Ober- und Unterkiefer zu klemmen, mit der die Kontaktpunkte und die beim Zubeißen auftretenden Kräfte unmittelbar gemessen werden können. Nachteilig ist jedoch, dass der durch die Dicke der Sensorfolie hervorgerufene Messfehler größer ist, als die verhältnismäßig geringe Amplitude der Zahnbewegungen beim Zubeißen. Wegen der Dicke der Sensorfolie sind die Zähne auch nicht in der natürlichen Okklusion. Die auf der Sensorfolie beaufschlagten Kontaktpunkte entsprechen also nicht der Realität. Hinzu kommt, dass die automatische Zuordnung der gemessenen Kontaktpunkte mit den einzelnen Zähnen und Zahnoberflächen nicht möglich ist. Eine solche Zuordnung ist jedoch für die Herstellung von Zahnersatz unabdingbar, weil nur so erkennbar ist, welche Teile der Zahngeometrie weggeschliffen oder geformt werden muss.

Ein weiterer Nachteil der Sensorfolie ist, dass sie nur die Kräfte messen kann, die senkrecht zur Sensorfolie gerichtet sind. Die restlichen Komponenten der tatsächlichen Kraftverteilung bleiben unbekannt. Zudem muss jede einzelne Folie ausgeglichen oder kalibriert werden, um korrekte relative oder absolute Ergebnisse der Kraftmessung zu erhalten. Im Übrigen kann eine Sensorfolie nur wenige Male verwendet werden.

Aufgabe der Erfindung ist es daher, ein einfach und kostengünstig umzusetzendes Verfahren vorzuschlagen, mit dem sich die Bewegungen einzelner Zähne unter Beaufschlagung durch die Beißkraft im tatsächlichen Zubiss und die Einflüsse der Bewegungen auf Kontaktpunkte und Kontaktflächen ermitteln lassen. Zudem ist es die Aufgabe, ein entsprechendes System zur Durchführung des Verfahrens zur Verfügung zu stellen.

Diese Aufgaben werden durch das Verfahren nach Anspruch 1 und das System nach Anspruch 9 gelöst. Bevorzugte Ausgestaltungen ergeben sich aus den jeweiligen Unteransprüchen.

Zusammengefasst liegt der Kern der Erfindung darin, dass zusätzlich zu den beiden Datensätzen enthaltend die dreidimensionalen Oberflächendaten von Ober- und Unterkiefer ein Satz von ebenfalls dreidimensionalen Außenflächendaten im Schlussbiss mithin unter Einwirkung der Beißkraft aufgenommen wird, wobei eine Teilfäche der Außenflächendaten für die Zuordnung von Ober- und Unterkiefer verwendet wird. Diese Außenflächendaten repräsentieren die durch die Krafteinwirkung bewegten Zähne. Der grundlegende Gedanke ist nun, die den Außenflächendaten inhärente Bewegung einzelner Zähne auf die Oberflächendaten von Ober- und Unterkiefer anzuwenden und diese entsprechend der erfolgten Bewegung zu modifizieren. Mit solchermaßen modifizierten Oberflächendaten lässt sich nun ein digitales Modell der Zähne im kraftbeaufschlagten Schlussbiss erstellen, aus dem sich die tatsächlichen Kontaktpunkte und Kontaktflächen ermitteln lassen. Dabei können die Oberflächendaten von Ober- und Unterkiefer unmittelbar am Patienten oder an einem vom Patienten abgenommenen Modell insbesondere mittels einer (Intraoral-)Kamera abgenommen werden.

Der wesentliche Gesichtspunkt der Erfindung ist somit, dass zumindest Teile der den Bukkalbiss repräsentierenden Außenflächendaten, die mit einer Intraoralkamera aufgenommen werden können, für die Registrierung der von Ober- und Unterkiefer vorhandenen Oberflächendaten genutzt werden. Dabei werden diejenigen Teile der Bukkalbissaufnahme für die Registrierung herangezogen, die durch die Krafteinwirkung nicht verformt wurden. So können der Oberkiefer und der Unterkiefer in einem ersten Schritt als Ganzes in die korrekte räumliche Lage zueinander gebracht werden. In einem zweiten Schritt werden nun die Teilflächen der Bukkalbissaufnahme betrachtet, deren Zähne durch die Krafteinwirkung verformt wurden. Durch die Anwendung einer solchen "nicht-rigiden" Transformation können die durch Kräfte verursachten Lageänderungen der einzelnen Zähne beim Zubeißen berücksichtigt werden. Dabei wird vorteilhafterweise eine Segmentierung der einzelnen Zähne vorgenommen, so dass die Zähne im digitalen Modell separat voneinander bewegt werden können.

Durch Übertragung dieser dem Bukkalbiss immanenten Verformung auf die einzelnen Zähne des Oberflächenmodells können die tatsächlichen Zahnpositionen und die Zahnausrichtungen im digitalen Modell berücksichtigt werden. Entsprechend können daraus die tatsächlichen Zahnkontakte mit den Kontaktflächen des Gegenkiefers berechnet werden. Mit den Ergebnissen, die sich aus der erfindungsgemäßen Vorgehensweise ergeben, kann darüber hinaus passgenauer Zahnersatz hergestellt werden.

Anspruchsgemäß wird das Verfahren so umgesetzt, dass zunächst im Schlussbiss unter der entsprechenden Einwirkung von Beißkräften der weitere Datensatz enthaltend die dreidimensionalen Außenflächendaten der Zähne, die in den von den Oberflächendaten erfassten Abschnitten der Zahnreihen liegen, angefertigt wird. In diesen Außenflächendaten werden dann im Oberkiefer und im Unterkiefer Registrierungsbereiche definiert, die im Schlussbiss keiner Krafteinwirkung ausgesetzt und demnach keiner Verformung ausgesetzt sind. Da diese Registrierungsbereiche entsprechend in den Oberflächendaten der nicht verformten Ober- und Unterkiefer vorhanden sind, können sie zur Registrierung dieser unterschiedlichen Datensätze herangezogen werden. Anhand dieser Registrierungsbereiche werden dann die ersten und die zweiten Oberflächendaten auf die Außenflächendaten registriert. Durch Vergleich von Strukturen in den Außenflächendaten und entsprechender Strukturen in den Oberflächendaten werden außerhalb der Registrierungsbereiche einzelne Zähne ermittelt, die im Schlussbiss eine Bewegung vollzogen haben. Diese Bewegungen werden dann in die Oberflächendaten transformiert, so dass unter Berücksichtigung der Bewegungen ein modifiziertes digitales Oberflächenmodell vom Schlussbiss erstellt werden kann.

Der wesentliche Vorteil der erfindungsgemäßen Vorgehensweise liegt darin, dass die zwischen den Zähnen auftretenden Kontaktpunkte und Kontaktflächen wesentlich besser berechnet werden können, als das mit den bislang bekannten Verfahren je möglich war. Ein weiterer Vorteil ist, dass den Schlussbiss keine zwischen den Zähnen eingeklemmte Sensorfolie verfälscht. Außerdem ist eine direkte Zuordnung der Kontaktpunkte zu der jeweiligen Zahngeometrie möglich. Da die Position der Kontaktpunkte auf der jeweiligen Zahngeometrie bekannt ist, können nicht nur die Kräfte senkrecht zu einer Sensorfolie sondern darüber hinaus auch die Richtung der Kraftvektoren bestimmt werden.

In einer besonders vorteilhaften Ausführungsform werden aufeinanderfolgende Bukkalbisse mit unterschiedlichen Beißkräften aufgenommen. Durch die Berücksichtigung von mehreren bukkalen Registraten bei der Registrierung können Bewegungssequenzen der Zähne erfasst und sich zeitlich verändernde Kontaktpunktemuster naturgetreu ermittelt werden. Aus den solchermaßen gemessenen (nicht-rigiden) Verformungen können auch Kraftberechnungen gespeist werden. So können beispielsweise Spannungskräfte berechnet werden, die die Einzelzahnbewegungen auf den Kieferknochen übertragen. Zudem können Kräfte zwischen einem Zahn und seinem periodontalen Ligament berechnet werden. Diese Kräfte können dann auf die Okklusionsseite übertragen und damit Länge und Richtung der Kraftvektoren auf der Okklusion bestimmt werden.

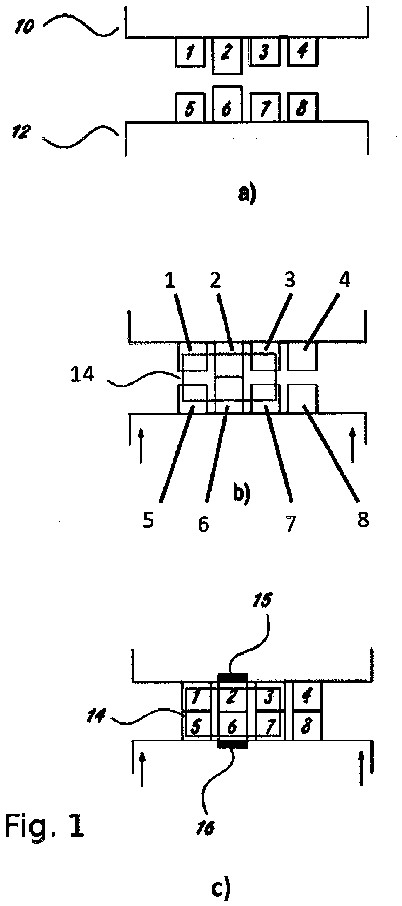

Nachfolgend wird die Erfindung anhand der Figuren 1a bis 1c und in mehreren Ausführungsbeispielen beschrieben:
Zunächst sei die erfindungsgemäße Vorgehensweise an einer ersten Ausführungsvariante erläutert: Bei dieser werden zur Erstellung der Oberflächendatensätze zunächst die Zahnreihen des Oberkiefers und des Unterkiefers eines Patienten mit einer Intraoralkamera zumindest im Rahmen der interessierenden Abschnitte abgescannt. Da das Scannen bei geöffnetem Mund respektive an den Kieferbereichen eines Modells geschieht, wirken in diesem Moment keine (Beiß-)Kräfte auf die Zähne. Vorteilhafterweise werden die einzelnen Zähne segmentiert, damit sie im digitalen Modell entsprechend separat voneinander bewegt werden können. So können nicht-rigide Bewegungen der einzelnen Zähne berücksichtigt werden.

Dieser erste Schritt der Oberflächenscans ist in Figur 1a gezeigt, wo der gescannte Oberkiefer 10 mit seinen Zähnen 1-4 sowie der gescannte Unterkiefer 12 mit seinen Zähnen 5-8 dargestellt ist. Dies ist die Situation, bei der keine Kräfte auf die Zähne wirken.

Figur 1b zeigt nun die Situation, in welcher der Patient leicht zubeißt, jedoch noch keine Beißkräfte auf die Zähne wirken. Allerdings treffen die Zähne 2 und 6 gerade soeben aufeinandertreffen, weil diese länger als die übrigen Zähne sind. Das mit unterbrochener Linie dargestellte Fenster 14 verdeutlicht den Bildausschnitt der Intraoralkamera, in der der Bukkalbiss "gesehen" werden kann.

Im nächsten Schritt nach Figur 1c beißt der Patient im Schlussbiss zu, wobei sich die Zähne 2 und 6 wegen der Beisskräfte in ihren Ligamenten verschieben respektive versinken. Die im Ligament versunkenen Bereiche 15 und 16 sind mit der Schraffur angedeutet. Die tatsächlichen Zahnpositionen weichen in diesem Stadium wegen der Verschiebungen von den in dem digitalen Modell manifestierten Positionen ab. In diesem festen Schlussbiss werden nun Außenflächendaten, mithin der Bukkalbiss, insbesondere mit einer Intraoralkamera aufgenommen. Die durch die Beißkräfte hervorgerufenen, nicht-rigiden Zahnbewegungen, sind damit in dem Bukkalbiss "verschlüsselt".

Als Registrierungsbereiche werden nun solche insbesondere automatisch ausgewählt, die im Bukkalbiss als solche identifiziert werden, die nicht von Beißkräften beaufschlagt sind und sich daher nicht bewegt haben. Diese Auswahl kann automatisch anhand eines Abgleiches von Oberflächendaten mit Außenflächendaten geschehen. Bereiche, die in beiden Datensätzen als identisch identifiziert werden, können als Registrierungsbereiche herhalten. In diesem Falle werden also nur die Teilbereiche der Zähne 1, 3, 5, und 7 für die Registrierung herangezogen.

Wegen der Segmentierung der Zähne im digitalen Modell können nun die aus dem nicht-rigiden Bukkalbiss ermittelten Bewegungen in einem weiteren Schritt auf die Einzelzähne im digitalen Modell übertragen werden. In diesem modifizierten digitalen Modell lassen sich Kontaktpunkte und Kontaktflächen zwischen den Zähnen ermitteln, die der tatsächlichen Situation im Mund des Patienten wesentlich stärker entsprechen, als es im anfänglichen rigiden digitalen Modell der Fall war.

Im vorliegenden Beispiel werden die Zähne 2 und 6 anhand der Segmentierung im digitalen Modell separat so an die Bukkalaufbissausnahme registriert, dass sie perfekt an dem Bukkalbiss zu liegen kommen. Damit ist auch bekannt, wie weit die Zähne 2 und 6 in die Ligamente eingetaucht sind, so dass die entsprechenden Kräfte berechnet werden können.

In einer zweiten Ausführungsvariante wird innerhalb der Schließbewegung eine Sequenz von Zuständen unter sich verändernden Krafteinwirkung aufgenommen. Zur Erstellung eines ersten digitalen Modells werden dazu wiederum zunächst die Zahnreihen von Ober- und Unterkiefer des Patienten mit einer Intraoralkamera abgescannt. Die einzelnen Zähne werden dann in dem digitalen Modell segmentiert. Danach wird die Intraoralkamera in eine Position gebracht, um den Bukkalbiss unter Einwirkung der Beißkraft aufzunehmen. Nun führt der Patient eine Schließbewegung durch, wobei zu Beginn der Schließbewegung keiner der Zähne in Kontakt mit seinem Gegenstück ist. Während der Schließbewegung, insbesondere nach der ersten Berührung der Zähne, werden zu bestimmten aufeinanderfolgenden Zeitpunkten jeweils Außenflächendaten mit der Intraoralkamera aufgenommen, so dass Bukkalaufnahmen für mehrere Zeitschritte der Schließsequenz unter zunehmender Kraftbeaufschlagung und entsprechend sich verändernder Zahnstellung vorliegen. Am Ende der Schließbewegungssequenz sind mehrere oder alle Zähne unter Bisskraft in Kontakt.

Die Registrierung greift auf eine Segmentierung der gescannten Zähne von Ober- und Unterkiefer zurück und wertet die Schließsequenz wie folgt aus:
Für jeden Zeitschritt der Schließsequenz wird eine Bukkalbiss-Registrierung des Oberkiefers mit dem Unterkiefer durchgeführt. Dabei werden in jedem Zeitschritt nur die Zähne für die Bukkalbiss-Registrierung als Registrierungsbereiche verwendet, die (noch) keinen Kontakt mit der Gegenseite haben und entsprechend nicht unter Kraftbeaufschlagung stehen. Dadurch ist sichergestellt, dass die Zähne für die Bukkalbiss-Registrierung verwendet werden, die keiner elastischen Deformation (im Ligament) unterworfen sind. Entsprechend wird für jeden Zeitschritt der Schließsequenz eine (rigide) Transformation Tᵢ, die die Lage vom Oberkiefer zum Unterkiefer beschreibt, erzeugt. Die Lage der nicht-rigiden, weil schon in Kontakt befindlichen, Zähne wird im Zeitschritt i durch Transformation erzeugt, bei der die Bewegungen einzelner Zähne aus den Außenflächendaten ermittelt und als entsprechende Bewegungen in die Oberflächendaten transformiert werden. Die so ermittelte finale Position und Orientierung eines Zahnes zum Gegenkiefer entspricht mit hoher Genauigkeit der Wirklichkeit.

Anhand der sich nicht berührenden Zähne kann die absolute Lage vom Unterkiefer zum Oberkiefer zu jedem Zeitpunkt ermittelt werden. Dadurch kann auch mittelbar eine Aussage über die Position und die Lage der Kiefergelenke gemacht werden, da die Aufnahme der absoluten Lage der Kiefer zueinander nicht durch lokal bewegte Zähne verfälscht wird. Mit der Registrierung gegen den Bukkalbiss kann entsprechend für die sich berührenden Zähne ermittelt werden, wie stark sich der jeweilige Zahn wegen der Krafteinwirkung im Ligament versenkt hat. Daraus ergeben sich unter Berücksichtigung der Verformungseigenschaften respektive der Materialparameter rechnerisch die im Ligament auftretenden Kräfte und entsprechend die an den Kontaktpunkten der Okklusion wirkenden Kräfte.

In einer zweiten Ausführungsvariante können Kontaktflächen und Kontaktkräfte ermittelt werden, auch wenn alle Zähne einer Krafteinwirkung unterliegen. Dazu werden wiederum zur Erstellung der dreidimensionalen Oberflächendatensätze die Zahnreihen von Ober- und Unterkiefer mit einer Intraoralkamera abgescannt. Mit diesen Scanns werden auch Bereiche der Gingiva von Ober- und Unterkiefer erfasst.

In diesem Fall werden die einzelnen Zähne im digitalen Modell zusammen mit der Gingiva segmentiert.

In einem weiteren Schritt wird die Intraoralkamera in die Bukkalbiss-Position gebracht, wobei das Sichtfeld der Kamera in diesem Fall nicht nur Zähne, sondern Zähne samt der Gingiva von Ober- und Unterkiefer erfasst. Dann führt der Patient eine Schließbewegung durch, zu deren Beginn keiner der Zähne in Kontakt mit der Gegenseite ist. Zum Ende der Schließbewegungssequenz sind alle von der Intraoralkamera erfassten Zähne unter Krafteinwirkung in Kontakt. Insbesondere versinken alle Zähne mehr oder weniger tief in ihrem Ligament. Nun greift der Registrierungsalgorithmus auf die Segmentierung zurück und wertet die Schließsequenz aus, indem nur die Gingiva des Ober- und Unterkiefers für die Registrierung der Lagebeziehung herangezogen werden. Über die Zahnbewegungen in Bezug zur (ortsfesten) Gingiva können die Kräfte zwischen allen Zähnen und dem Ligament berechnet werden, indem sie aus den Versenkungsamplituden der Zähne im Ligament und den mechanischen Eigenschaften der Ligamente berechent werden. Die Berechnung der auftretenden Kräfte kann beispielsweise durch eine Finite-Elemente-Methode erfolgen.

Alternativ kann die zweite Ausführungsvariante dazu eingesetzt werden, die beim Zubiss tatsächlich auftretenden Kräfte mit Hilfe einer Sensorfolie in absoluten Werten zu ermitteln. Dazu wird eine Sensorfolie zwischen die Zähne gebracht, die eine Messung der absoluten Kräfte erlaubt. Nun wird mit einer Intraoralkamera eine Schließsequenz mit Sensorfolie als Bukkalbiss-Sequenz aufgenommen. Wiederum werden die segmentierten Einzelzähne des digitalen Zahnmodells mit der Bukkalbiss-Sequenz in Deckung gebracht. Die von der Folie aufgenommenen Kontaktpunkte werden mit den korrekt ausgerichteten Zähnen zur Deckung gebracht. Dieselbe Schließsequenz wird auch ohne Sensorfolie aufgenommen. Im Falle beider Schließsequenzen wird die erfindungsgemäße Registrierung auf das Oberflächenmodell angewandt, um die natürlichen Zahnstellungen mit und ohne Folie zu ermitteln.

In Kenntnis des tatsächlichen Schlussbisses ohne die Sensorfolie können die mit der Sensorfolie gemessenen Kräfte auf die Situation ohne Folie übertragen werden. Die Verfälschung durch die Dicke der Sensorfolie kann somit herausgerechnet werden. Die in der Sensorfolie verbleibende Verformung kann nach der Kraftmessung mit der Intraoralkamera gescannt werden. Durch die Aufnahme der räumlichen Verwerfung der Sensorfolie kann die Genauigkeit der Zuordnung weiter verbessert werden. Die so aufgenommene räumliche Verwerfung kann ihrerseits mit den Zahngeometrien in Bezug gesetzt mithin registriert werden und somit die Zuordnung von Kontaktpunkten auf der Folie mit Kontaktpunkten auf den Zahngeometrien ermittelt werden.

Alternativ bietet es sich an, die Sensorfolie in einer definierten Weise mechanisch mit der Intraoralkamera zu verbinden. Durch Kenntnis der mechanischen Kupplung zwischen Sensorfolie und Intraoralkamera können die gemessenen Kontaktpunkte einfacher auf die Zahngeometrien übertragen werden, da so ein gemeinsames Koordinatensytem zwischen der Sensorfolie und den aufgenommenen Zahngeometrien geschafften wird.

Vorteilhafterweise wird die Sensorfolie mit einem definierten Muster bedruckt, das die Zuordnung zwischen den aufgenommenen Kontaktpunkten und den Zahngeometrien erleichtert. Die Zuordnung geschieht, indem ein Teil der Muster bei der BukkalbissAufnahme mit erfasst wird und die räumliche Zuordnung zwischen Folie und Zahngeometrien zumindest teilweise bekannt ist.

Statt einer Sensorfolie kann ein herkömmliches Blaupausen-Papier verwendet werden. Nach der Schlussbiss-Aufnahme mit dem Blaupausenpapier und der Bukkalaufnahme wird das Blaupausenpapier mit der Intraoralkamera aufgenommen und die Kontaktpunkte aus der Schlussbiss-Position ermittelt. Nun werden die auf dem Blaupausenpapier ersichtlichen Kontaktpunkte auf die tatsächlichen Zahngeometrien übertragen. Zudem kann die auf den Zähnen abgedrückte Blaupausenfarbe mit einem weiteren Scan mit der Intraoralkamera erfasst werden. Dadurch wird die Zuordnung zwischen den Blaupausenpunkten auf dem Papier und den korrespondierenden Punkten vereinfacht.

Es kann auch vorteilhaft sein, das Blaupausenpapier in einer definierten Weise mechanisch mit der Intraoralkamera zu verbinden. Durch die Kenntnis der mechanischen Kupplung zwischen Blaupausenpapier und Intraoralkamera können die gemessenen Kontaktpunkte einfacher auf die Zahngeometrien übertragen werden, indem so ein gemeinsames Koordinatensystem zwischen Kontaktpunkten auf dem Blaupausenpapier und den aufgenommenen Zahngeomtrien geschaffen wird.

Wie auch die oben beschriebene Sensorfolie, so kann auch das Blaupausenpapier zusätzlich mit einem definierten Muster bedruckt werden.

In einer besonderen Ausführungsform wird die im Bukkalbiss errechnete Lage eines Zahnes zur Berechnung der auf den Zahn wirkenden Kraft verwendet, wobei insbesondere bestimmte Zähne durch Registrate beim Zubeissen aus der Okklusion genommen werden. Solche Registrate können auf der Basis von Kieferbewegungsdaten gedruckt werden. Dabei ist es auch vorteilhaft, die (gedruckten) Registrate in verschiedenen Winkelungen anzufertigen, um bestimmte Teile oder Richtungen des periodontalen Ligaments zu belasten und damit Kräfte zu berechnen.

In einer weiteren vorteilhaften Ausführungsform können die zumindest teilweise nicht-rigiden Transformationen auf die Zahnwurzeln einer passenden tomographischen Aufnahme des Knochens übertragen werden. Auch können die Kräfte an den Zahnwurzeln und dem umgebenden Gewebe berechnet werden.

## Patentansprüche

1. Computerimplementiertes Verfahren zur Darstellung einer Zahnstellung unter Einwirkung von Beißkräften,
wobei erste Oberflächendaten (10) von zumindest einem Abschnitt der Zahnreihe
des Oberkiefers und zweite Oberflächendaten (12) von zumindest einem gegenüberliegenden Abschnitt der Zahnreihe des Unterkiefers angefertigt werden,
wobei aus den Oberflächendaten ein digitales Oberflächenmodell erzeugt wird,
wobei unter Einwirkung von Beißkräften im Schlussbiss ein Datensatz enthaltend Außenflächendaten von Zähnen, die in den Abschnitten der Zahnreihen liegen, angefertigt wird,
wobei in den Außenflächendaten Registrierungsbereiche im Oberkiefer und im Unterkiefer definiert werden, die im Schlussbiss keiner Krafteinwirkung ausgesetzt sind,
wobei anhand der Registrierungsbereiche die ersten und die zweiten Oberflächendaten auf die Außenflächendaten registriert werden,
wobei außerhalb der Registrierungsbereiche Bewegungen (15, 16) einzelner Zähne
aus den Außenflächendaten ermittelt und die Bewegungen in die Oberflächendaten transformiert werden und
wobei unter Berücksichtigung der Bewegungen ein modifiziertes digitales Oberflächenmodell erstellt wird.

2. Computerimplementiertes Verfahren nach Anspruch 1, **dadurch gekennzeichnet,**
**dass** aus den Oberflächendaten einzelne Zähne segmentiert und die Bewegungen auf ein modifiziertes digitales Oberflächenmodell enthaltend die segmentierten Zähne übertragen werden.

3. Computerimplementiertes Verfahren nach Anspruch 1 oder 2, **dadurch gekennzeichnet,**
**dass** aus dem modifizierten digitalen Oberflächenmodell Zahnkontakte mit Kontaktpunkten und/oder Kontaktflächen und/oder Kontaktdistanzen und/oder Kontaktkräften berechnet werden.

4. Computerimplementiertes Verfahren nach einem der vorherigen Ansprüche, **dadurch gekennzeichnet,**
**dass** in den Außenflächendaten als Registrierungsbereiche Zähne definiert werden, die nicht im Kontakt mit dem gegenüberliegenden Zahn stehen.

5. Computerimplementiertes Verfahren nach einem der vorherigen Ansprüche, **dadurch gekennzeichnet,**
**dass** in den Außenflächendaten als Registrierungsbereiche Bereiche des Zahnfleisches definiert werden.

6. Computerimplementiertes Verfahren nach einem der vorherigen Ansprüche, **dadurch gekennzeichnet,**
**dass** die Registrierungsbereiche mit einem Registrieralgorithmus automatisch definiert werden.

7. Computerimplementiertes Verfahren nach einem der vorherigen Ansprüche, **dadurch gekennzeichnet,**
**dass** innerhalb der Schließbewegung eine Sequenz von Zuständen unter sich verändernden Krafteinwirkung aufgenommen werden, wobei zu bestimmten aufeinanderfolgenden Zeitpunkten jeweils Außenflächendaten aufgenommen werden.

8. Computerimplementiertes Verfahren nach einem der vorherigen Ansprüche,
**dadurch gekennzeichnet,**
**dass** die im Bukkalbiss errechnete Lage eines Zahnes zur Berechnung der auf den Zahn wirkenden Kraft verwendet wird, wobei insbesondere bestimmte Zähne durch Registrate beim Zubeissen aus der Okklusion genommen werden.

9. System zur Datenverarbeitung, umfassend Mittel zur Umsetzung des Verfahrens nach einem der vorherigen Ansprüche.

## Claims

1. A computer-implemented method for representing a tooth position under the effect of biting forces, wherein first surface data (10) of at least one section of a row of teeth of the maxilla and second surface data (12) of at least one opposing section of a row of teeth of the mandible are generated, wherein a digital surface model is produced from the surface data,
wherein a data set containing outer surface data of teeth which lie in the sections of the rows of teeth under the action of biting forces in occlusion is generated,
wherein registration regions in the maxilla and in the mandible are defined in the outer surface data, the registration regions not being exposed to the action of any force in occlusion,
wherein the first and the second surface data are registered onto the outer surface data with the aid of the registration regions,
wherein movements (15, 16) of individual teeth outside the registration regions are determined from the outer surface data, and the movements are transformed into the surface data, and
wherein a modified digital surface model which takes the movements into account is generated.

2. The computer-implemented method as claimed in claim 1, **characterized in that** individual teeth are segmented from the surface data and the movements are transferred to a modified digital surface model containing the segmented teeth.

3. The computer-implemented method as claimed in claim 1 or claim 2, **characterized in that** tooth contacts with contact points and/or contact surfaces and/or contact distances and/or contact forces are calculated from the modified digital surface model.

4. The computer-implemented method as claimed in one of the preceding claims, **characterized in that** teeth which are not in contact with the opposing tooth are defined as registration regions in the outer surface data.

5. The computer-implemented method as claimed in one of the preceding claims, **characterized in that** regions of the gingiva are defined as registration regions in the outer surface data.

6. The computer-implemented method as claimed in one of the preceding claims, **characterized in that** the registration regions are automatically defined using a registration algorithm.

7. The computer-implemented method as claimed in one of the preceding claims, **characterized in that** a sequence of states is recorded under the action of varying forces within the closing movement, wherein the respective outer surface data are recorded at specific successive times.

8. The computer-implemented method as claimed in one of the preceding claims, **characterized in that** the position of a tooth computed in the buccal bite is used to calculate the force acting on the tooth, wherein in particular, specific teeth are excluded from the occlusion when biting together by means of registrations.

9. A data processing system comprising means for implementation of the method as claimed in one of the preceding claims.

## Revendications

1. Procédé informatisé pour représenter une position de dents sous l'effet de forces occlusales, dans lequel des premières données de surface (10) d'au moins un tronçon de la rangée de dents de la mâchoire supérieure et des secondes données de surface (12) d'au moins un tronçon opposé de la rangée de dents de la mâchoire inférieure sont réalisées, dans lequel un modèle de surface numérique est produit à partir des données de surface,
dans lequel sous l'action de forces occlusales dans l'occlusion, un jeu de données contenant des données de surface extérieure de dents qui sont placées dans les tronçons des rangées de dents, est effectué,
dans lequel dans les données de surface extérieure, des zones de marquage d'articulation dans la mâchoire supérieure et dans la mâchoire inférieure sont définies, qui ne sont soumises à aucune force occlusale dans l'occlusion,
dans lequel à l'aide des zones de marquage d'articulation, les premières et les secondes données de surface sont marquées sur les données de surface extérieure,
dans lequel en-dehors des zones de marquage d'articulation, des mouvements (15, 16) de dents individuelles sont déterminés à partir des données de surface extérieure et
dans lequel en tenant compte des mouvements, un modèle de surface numérique modifié est réalisé.

2. Procédé informatisé selon la revendication 1, **caractérisé en ce qu'**à partir des données de surface, des dents individuelles sont segmentées et les mouvements sont transmis sur un modèle de surface numérique modifié contenant les dents segmentées.

3. Procédé informatisé selon la revendication 1 ou 2, **caractérisé en ce qu'**à partir du modèle de surface numérique modifié, des contacts dentaires avec des points de contact et/ou des surfaces de contact et/ou des distances de contact et/ou des forces de contact sont calculés.

4. Procédé informatisé selon l'une des revendications précédentes, **caractérisé en ce que** dans les données de surface extérieure, des dents qui sont ne sont pas en contact avec la dent opposée sont définies en tant que zones de marquage d'articulation.

5. Procédé informatisé selon l'une des revendications précédentes, **caractérisé en ce que** dans les données de surface extérieure, des zones de la gencive sont définies en tant que zones de marquage d'articulation.

6. Procédé informatisé selon l'une des revendications précédentes, **caractérisé en ce que** les zones de marquage d'articulation sont définies automatiquement avec un algorithme de marquage d'articulation.

7. Procédé informatisé selon l'une des revendications précédentes, **caractérisé en ce qu'**à l'intérieur du mouvement de fermeture, une séquence d'états sous effet de la force en évolution est enregistrée, dans lequel à des moments consécutifs définis, des données de surface extérieure respectives sont enregistrées.

8. Procédé informatisé selon l'une des revendications précédentes, **caractérisé en ce que** la position d'une dent calculée dans l'occlusion buccale est utilisée pour calculer la force agissant sur la dent, dans lequel, en particulier, certaines dents sont sorties de l'occlusion par des papiers à articuler lors de l'occlusion.

9. Système de traitement de données, comprenant des moyens pour mettre en œuvre le procédé selon l'une des revendications précédentes.
